# EUROPEAN PATENT APPLICATION

(11) **EP 4 813 935 A1**
(43) Date of publication of application: **30.09.2026**
(21) Application number: 25901630.1
(22) Date of filing: 06.08.2025
(51) Int. Cl.: C02F 9/00, C02F 1/02, C02F 1/52, C02F 1/66, C02F 1/00, C02F 1/58, C02F 1/68, C01D 5/16, C22B 3/20, A61L 9/01

(54) **METHOD FOR PREPARING SODIUM SULFATE DECAHYDRATE CRYSTALS FROM HIGH-CONCENTRATION SALT WASTEWATER OF SECONDARY BATTERY BY AQUEOUS SOLUTION CRYSTAL GROWTH METHOD, AND RECYCLING METHOD USING SAME**

(30) Priority: 04.12.2024 KR 20240178799
(71) Applicant: Kari Co., Ltd., Hwaseong-si, Gyeonggi-do 18545 (KR)
(72) Inventor: KIM, Pan Chae, Daegu 42769 (KR); KIM, Seon Won, Daegu 41121 (KR); YANG, Hee Gyoung, Seoul 06650 (KR); LEE, Dong Seong, Hwaseong-si, Gyeonggi-do 18546 (KR); LEE, Soo Jin, Seoul 06976 (KR); JEONG, Min Chang, Incheon 21997 (KR); JEONG, Seong Kyu, Yongin-si, Gyeonggi-do 17148 (KR); JEONG, Kyung Sub, Iksan-si, Jeonbuk-do 54628 (KR); LEE, Han Kyung, Seoul 03488 (KR); KIM, Hee Jung, Daegu 42274 (KR); KANG, Sang Do, Daegu 42664 (KR)
(74) Representative: K&L Gates LLP
(86) International application number: PCT/KR2025/011788
(87) International publication number: WO 2026/121461

(57) **Abstract**

The present invention relates to a method for producing sodium sulfate decahydrate crystals from high-concentration secondary battery salt wastewater by an aqueous solution crystal growth method, and a resource recovery method using the same, more particularly including: Stage 1 of transporting high-concentration secondary battery salt wastewater to a preheater; Stage 2 of heating the salt wastewater introduced into the preheater to 30 to 40 °C by supplying high-temperature steam (high-temperature vapor) discharged from a hydrothermal reactor to the preheater; Stage 3 of transporting the salt wastewater preheated in stage 2 to a horizontal pipe-type hydrothermal reactor and then performing a hydrothermal reaction that heats the salt wastewater to 120 to 150°C for 30 to 60 minutes in order to evaporate moisture until a salinity of the introduced salt wastewater reaches 8 to 9%, thereby producing a sodium sulfate supersaturated solution; Stage 4 of feeding the high-temperature steam discharged in stage 3 to the preheater in stage 2, and transporting the sodium sulfate supersaturated solution with the salinity of 8 to 9% to a supersaturated solution storage tank; Stage 5 of supplying carbon dioxide to the sodium sulfate supersaturated solution flowing into the supersaturated solution storage tank in order to adjust the pH to a range of 7 to 7.5 while precipitating nickel carbonate (NiCO₃), followed by separating and recovering the precipitate so as to remove nickel heavy metals contained in the sodium sulfate supersaturated solution; Stage 6 of transporting the sodium sulfate supersaturated solution with the pH adjusted to 7 to 7.5 and carbon dioxide to an aqueous solution crystal growth tank; Stage 7 of performing a vertical temperature gradient method, which controls an upper temperature of the aqueous solution crystal growth tank to a temperature in the range of 15 to 20 °C and a lower temperature thereof to a temperature in the range of 2 to 4 °C, for 12 to 24 hours, thereby growing and obtaining sodium sulfate decahydrate crystals; Stage 8 of filtering and separating the sodium sulfate decahydrate crystals obtained in stage 7 using a 60 to 100 mesh net and recovering the remaining filtrate; Stage 9 of naturally drying the sodium sulfate decahydrate crystals filtered and separated in stage 8; and Stage 10 of mixing the steam discharged from the preheater in stage 2 with the filtrate recovered by filtering and separating the sodium sulfate decahydrate crystals in stage 8, so as to produce usable water to be reused in a resource recovery process.

## Description

### [Field of Invention]

The present invention relates to a technology regarding appropriately treating and recycling high-concentration salt wastewater discharged from a precursor production process for secondary battery cathode materials, in particular, to the preparation of sodium sulfate decahydrate crystals from high-concentration secondary battery salt wastewater by a method for aqueous solution crystal growth so as to appropriately treat high-concentration salt wastewater, and in addition, to create various added values using byproducts generated in the above process.

### [Background of Invention]

Looking into the origin of generation of high-concentration salt wastewater from secondary batteries ('high-concentration secondary battery salt wastewater'), the demand for secondary batteries required for electric vehicles, etc. has rapidly increased since the 2000s, and relevant industries for battery components such as precursors, cathode materials (positive electrode active materials), anode materials, and electrolytes also have developed rapidly.

A precursor for cathode materials, which is the starting point of the battery industry, is produced by a co-precipitation method using a mixture of nickel sulfate (containing 80 to 90 wt.%), cobalt sulfate and manganese sulfate, ammonia as a complexing agent, and sodium hydroxide for pH adjustment. A filtrate discharged after solid-liquid separation is a high-concentration salt wastewater containing serious pollutants such as sulfate ions, salts, and nickel heavy metal.

In the case of high-concentration salt wastewater corresponding to the treatment target water, it has a sulfate ion concentration of generally 60,000 to 68,000 mg/L, salinity of 5 to 6%, pH 9 to 10, nickel (Ni) heavy metal content of 400 to 450 mg/L, and ecotoxicity value (Toxic Unit) TU 8 or higher. However, looking into the discharge standards for water pollutants (related to Article 34) in the Enforcement Rule of the Water Environment Conservation Act [Appendix 13], the discharge water quality standard is an ecotoxicity value of TU 1 or lower, and therefore, a treatment method satisfying these standards is required.

In order to purify the treatment target water to an ecotoxicity value of TU 1 or lower, a technology to remove sulfate ions, salts, nickel heavy metal, etc. is required. In particular, in the case of nickel heavy metal, which have the greatest impact on ecotoxicity, it is reported that nickel in the final treatment water should be 7.2 mg/L or less (removal rate of 98% or higher), and sulfate should be 4,605 mg/L or less (removal rate of 92% or higher).

If sodium sulfate decahydrate crystals are obtained in a large quantity from the high-concentration salt wastewater as the treatment target water, the concentrations of sulfate ions and salts contained in the high-concentration salt wastewater is reduced, so as not only to achieve water purification effects, but also to enable resource recovery if sodium sulfate decahydrate crystals are used as a raw material, thereby contributing to profit creation.

### [Prior Art Literature]

Korean Patent Registration No. 10-2169490
Korean Patent Registration No. 10-2613721
Korean Patent Laid-Open Publication No. 10-2021-0134544

### [Summary of Invention]

### [Technical Problem to be Solved]

The present invention was made in accordance with the above necessity, and the objects thereof are as follows.

First, it is an object of the present invention to provide a novel method for producing sodium sulfate decahydrate crystals from high-concentration secondary battery salt wastewater while simultaneously producing nickel carbonate powder, by means of a vertical temperature gradient method based on an aqueous solution crystal growth method.

Second, another object of the present invention is to provide a method for resource recovery capable of creating new added value, which includes preparing a valuable metal extractant, nickel sulfate powder, lithium carbonate powder, a desalinating agent for salt removal, an ammonia or hydrogen sulfide deodorant for odor removal, a color and heavy metal remover for dyeing wastewater, sodium sulfate powder, and an ammonia nitrogen remover for water purification using sodium sulfate powder, by applying sodium sulfate decahydrate crystals as described above.

Third, a further object of the present invention is to recycle the filtrate recovered after separating sodium sulfate decahydrate crystals, as the usable water required in a resource recovery process.

### [Technical Solution]

The present invention has the following configurations to achieve the above objects.

The present invention relates to a method for producing sodium sulfate decahydrate crystals from high-concentration secondary battery salt wastewater by an aqueous solution crystal growth method, and a resource recovery method using the same, more particularly, which comprises: Stage 1 of transporting high-concentration secondary battery salt wastewater to a preheater; Stage 2 of heating the salt wastewater introduced into the preheater to 30 to 40 °C by supplying high-temperature steam (high-temperature vapor) discharged from a hydrothermal reactor to the preheater; Stage 3 of transporting the salt wastewater preheated in stage 2 to a horizontal pipe-type hydrothermal reactor and then performing a hydrothermal reaction that heats the salt wastewater to 120 to 150 °C for 30 to 60 minutes in order to evaporate moisture until the salinity of the introduced salt wastewater reaches 8 to 9%, thereby producing a sodium sulfate supersaturated solution; Stage 3 of feeding the high-temperature steam discharged in the third step to the preheater in stage 2, and transporting the sodium sulfate supersaturated solution with the salinity of 8 to 9% to a supersaturated solution storage tank; Stage 5 of supplying carbon dioxide to the sodium sulfate supersaturated solution flowing into the supersaturated solution storage tank in order to adjust the pH to a range of 7 to 7.5 while precipitating nickel carbonate (NiCO₃), followed by separating and recovering the precipitate so as to remove nickel heavy metals contained in the sodium sulfate supersaturated solution; Stage 6 of transporting the sodium sulfate supersaturated solution with the pH adjusted to 7 to 7.5 and carbon dioxide to an aqueous solution crystal growth tank; Stage 7 of performing a vertical temperature gradient method, which controls an upper temperature of the aqueous solution crystal growth tank to a temperature in the range of 15 to 20 °C and a lower temperature thereof to a temperature in the range of 2 to 4 °C, for 12 to 24 hours, thereby growing and obtaining sodium sulfate decahydrate crystals; Stage 8 of filtering and separating the sodium sulfate decahydrate crystals obtained in stage 7 using a 60 to 100 mesh net and recovering the remaining filtrate; Stage 9 of naturally drying the sodium sulfate decahydrate crystals filtered and separated in stage 8; and Stage 10 of mixing the steam discharged from the preheater in stage 2 with the filtrate recovered by filtering and separating the sodium sulfate decahydrate crystals in stage 8, so as to produce usable water to be reused in a resource recovery process.

### [Effect of Invention]

The technical effects according to the configuration of the present invention are as follows.

First, using a vertical temperature gradient method based on an aqueous solution crystal growth method, sodium sulfate decahydrate crystals can be obtained from high-concentration secondary battery salt wastewater, and nickel carbonate powder can be obtained at the same time, thereby effectively removing serious environmental pollutants such as sulfate ions and nickel heavy metal contained in the high-concentration secondary battery salt wastewater.

Second, sodium sulfate decahydrate crystals obtained as a byproduct during the process of treating high-concentration secondary battery salt wastewater can be recycled as various resources to create new added value.

Third, by recycling the filtrate recovered after separating the sodium sulfate decahydrate crystals as the usable water required in a resource recovery process, an amount of the filtrate discharged to the outside can be minimized, and through this, environmental problems caused by high-concentration secondary battery salt wastewater can be drastically improved. In other words, even the filtrate from which environmental pollutants have been effectively removed is recycled as the usable water required in the resource recovery process instead of being discharged as it is, thereby fundamentally solving environmental problems.

### [Brief Description of Drawings]

FIG. 1 is a block diagram illustrating a process for producing sodium sulfate decahydrate crystals from high-concentration secondary battery salt wastewater by an aqueous solution crystal growth method according to the present invention, which includes processes from stage 1 to stage 10 of the present invention.
FIG. 2 shows the results of X-ray diffraction analysis for nickel carbonate powder obtained through a carbonation reaction of a sodium sulfate supersaturated solution.
FIG. 3 shows sodium sulfate decahydrate crystals produced using a vertical temperature gradient method based on an aqueous solution crystal growth method.
FIG. 4 is a block diagram illustrating a resource recovery process using sodium sulfate decahydrate crystals obtained according to the present invention, which includes processes from stage 11 to stage 22 of the present invention.
FIG. 5 shows that a cathode active material deposited on the surface of a waste crucible after calcination of the cathode active material, the cathode active material deposited on the surface of a waste crucible is peeled off and extracted by a valuable metal extractant is peeled off and extracted.
FIG. 6 shows the results of X-ray diffraction analysis for nickel sulfate powder obtained using an extract obtained from the waste crucible by a valuable metal extractant.
FIG. 7 shows the results of X-ray diffraction analysis for lithium carbonate powder obtained using an extract obtained from the waste crucible by a valuable metal extractant.
FIG. 8 shows a photograph of the results of cultivating onions as a cash crop after desalination using a desalinating agent for salt removal at the Saemangeum reclaimed land site.
FIG. 9 shows a process of reducing the color of dyeing wastewater.
FIG. 10 shows the results of X-ray diffraction analysis for sodium sulfate powder obtained by drying sodium sulfate decahydrate crystals at a high temperature.
FIG. 11 shows an ammonia nitrogen remover (carrier type) for water purification.

### [Preferred Embodiments for Carrying out the Invention]

Hereinafter, specific embodiments of the present invention will be described in more detail with reference to the accompanying drawings.

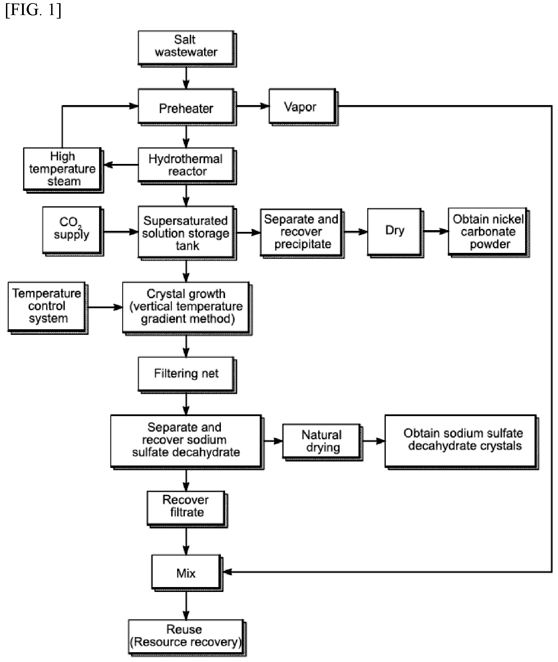

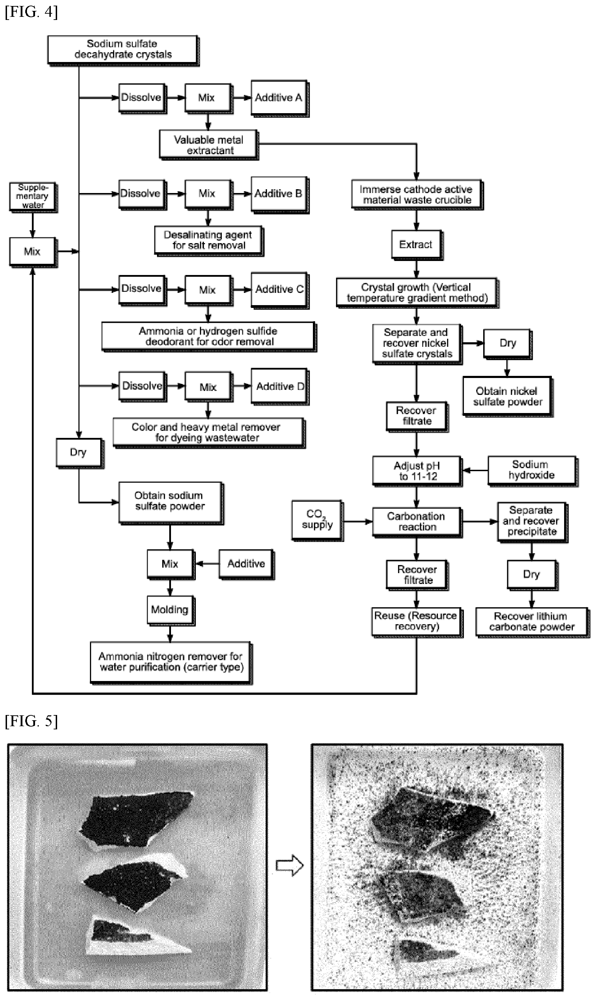

The present invention relates to the preparation of sodium sulfate decahydrate crystals from high-concentration secondary battery salt wastewater by an aqueous solution crystal growth method, as well as a resource recovery method using the same. In the block diagrams of FIGS. 1 and 4, the step-by-step processes are illustrated.

### (1) Stage 1

This is a process of transporting high-concentration secondary battery salt wastewater to a preheater.

High-concentration secondary battery salt wastewater is characterized by sulfate ion concentration of 60,000 to 68,000 mg/L, salinity of 5 to 6%, pH of 9 to 10, nickel (Ni) heavy metal content of 400 to 450 mg/L, and ecotoxicity value (Toxic Unit) of TU 8 or higher, and is stored in a storage tank and then transferred to the preheater.

### (2) Stage 2

This is a process of feeding high-temperature steam (high-temperature vapor) discharged from a hydrothermal reactor to the preheater and heating the salt wastewater flowing into the preheater to 30 to 40 °C.

A steam pipe is installed inside the preheater to feed the high-temperature steam (high-temperature vapor) discharged from the hydrothermal reactor to the steam pipe, and the salt wastewater flowing into the preheater is naturally preheated to 30 °C or higher as it passes through the preheater.

### (3) Stage 3

This is a process for producing a sodium sulfate supersaturated solution through a hydrothermal reaction in which the salt wastewater preheated in stage 2 is transported to a horizontal pipe-type hydrothermal reactor and then heated to 120 to 150 °C for 30 to 60 minutes to evaporate moisture until the salinity of the introduced salt wastewater reaches 8 to 9%.

At this time, the salinity % of the salt wastewater refers to the mass (g) of sodium sulfate contained in 100 ml of the salt wastewater. For example, if 8 g of sodium sulfate is contained in 100 ml of the salt wastewater, the salinity of the salt wastewater is 8%, and the salinity of the salt wastewater described in the claims of the present invention and the salinity of the salt wastewater described in the remainder of the specification are all calculated in the same manner.

The salt wastewater preheated while passing through the preheater is introduced into the horizontal pipe-type hydrothermal reactor at a high temperature of 120 to 150 °C, and then, a hydrothermal reaction is induced to quantitatively evaporate water until the salinity reaches 8 to 9% over a short period of 30 to 60 minutes, thereby producing a sodium sulfate supersaturated solution.

### (4) Stage 4

This is a process of circulating and feeding the high-temperature steam discharged in the stage 3 to the preheater in stage 2, and transporting the sodium sulfate supersaturated solution with a salinity of 8 to 9% to a supersaturated solution storage tank.

### (5) Stage 5

This is a process for removing nickel heavy metal contained in a sodium sulfate supersaturated solution by supplying carbon dioxide (CO₂) to the sodium sulfate supersaturated solution that has been introduced into the supersaturated solution storage tank to adjust the pH to a range of 7 to 7.5, precipitating nickel carbonate (NiCO₃), and then separating and recovering the precipitate.

The nickel heavy metal is removed through a separate process that supplies carbon dioxide to a sodium sulfate supersaturated solution having a pH range of 9 to 10, which was introduced into the supersaturated solution storage tank, to adjust the pH to a range of 7 to 7.5 and precipitate nickel carbonate (NiCO₃), followed by separating and recovering the precipitate.

### (6) Stage 6

This is a process of transporting the sodium sulfate supersaturated solution with a pH adjusted to 7 to 7.5 and carbon dioxide to an aqueous solution crystal growth tank.

A fluid, in which the sodium sulfate supersaturated solution (liquid) and carbon dioxide (gas) coexist, is introduced into the aqueous solution crystal growth tank.

### (7) Stage 7

This is a process of performing a vertical temperature gradient method, which controls an upper temperature of the aqueous solution crystal growth tank to a temperature in the range of 15 to 20 °C and a lower temperature thereof to a temperature in the range of 2 to 4 °C, for 12 to 24 hours, so as to grow and obtain sodium sulfate decahydrate crystals from a fluid.

The fluid with good fluidity introduced into the aqueous solution crystal growth tank is in a state where sodium ions and sulfate ions diffuse well, and sodium sulfate decahydrate crystals are grown using the vertical temperature gradient method.

A temperature control system is operated to maintain a temperature of an upper part of the aqueous solution crystal growth tank in the range of 15 to 20 °C, and a temperature of a lower part thereof in the range of 2 to 4 °C, wherein a temperature gradient (Δt) between the upper and lower parts of the aqueous solution crystal growth tank is set to 11 to 18 °C.

When a vertical temperature gradient is formed in this way, sodium ions and sulfate ions diffuse well up and down. This state is maintained for 12 to 24 hours to obtain sodium sulfate decahydrate crystals.

When the vertical temperature gradient method (VTG) based on an aqueous solution crystal growth method was used to produce sodium sulfate decahydrate crystals, a yield of sodium sulfate decahydrate crystals was about 5 to 10 wt.% higher than that of the conventional horizontal temperature reduction method (HTD). This is because the diffusion of sodium ions and sulfate ions occurred well and the dissolution and precipitation reaction mechanism proceeded actively.

### (8) Stage 8

This is a process of filtering and separating the sodium sulfate decahydrate crystals obtained in stage 7 using a 60 to 100 mesh net and recovering the remaining filtrate.

Sodium sulfate decahydrate crystals are filtered and separated using a mesh net, and the remaining filtrate is recovered. The filtrate is cooled in the aqueous solution crystal growth tank to become cold water at a temperature of 10 °C or less.

### (9) Stage 9

This is a process for obtaining sodium sulfate decahydrate crystals by naturally drying the sodium sulfate decahydrate crystals filtered and separated in stage 8.

### (10) Stage 10

This is a process of mixing the steam discharged from the preheater in stage 2 with the filtrate recovered by filtering and separating the sodium sulfate decahydrate crystals in stage 8, so as to produce usable water to be reused in the resource recovery process.

### (11) Stage 11

This is a process for obtaining sodium sulfate powder by drying the sodium sulfate decahydrate crystals obtained in stage 9 at a high temperature in a range of 200 to 300 °C for 20 to 30 minutes.

### (12) Stage 12

This is a process of adding 15 to 20 parts by weight (wt. parts) of the usable water prepared in stage 10 and 20 to 30 wt. parts of an additive (E) to 70 to 80 wt. parts of the sodium sulfate powder obtained in stage 11, and mixing them.

In this regard, the additive (E) is prepared by mixing 10 to 15 wt. parts of non-swelling mica powder, 5 to 7 wt. parts of calcium aluminate powder, 3 to 5 wt. parts of nanocarbon colloidal solution, and 2 to 3 wt. parts of silver (Ag) colloidal solution.

### (13) Stage 13

This is a process of molding the mixture in a slurry state obtained in stage 12.

Molding is performed using various molds.

### (14) Stage 14

This is a process of producing an ammonia nitrogen remover for water purification in various forms by naturally drying a molded product formed using the mold.

### (15) Stage 15

This is a process for producing a valuable metal extractant by dissolving 10 kg of sodium sulfate decahydrate crystals obtained in stage 9 in 20 L of the usable water prepared in stage 10 and then adding an additive (A) thereto.

In this regard, the additive (A) is prepared by mixing 0.5 L of a 35% concentration hydrochloric acid solution and 0.06 kg of ethylene diamine tetraacetic acid (EDTA).

At this time, the % concentration of the hydrochloric acid solution means the mass (g) of hydrochloric acid contained in 100 g of hydrochloric acid solution in which water and hydrochloric acid are mixed. For example, if 65 g of water and 35 g of hydrochloric acid are mixed to produce 100 g of hydrochloric acid solution, the hydrochloric acid solution becomes 35% concentrated. The % concentration of the hydrochloric acid solution described in the claims of the present invention and the % concentration of the hydrochloric acid solution described in the remaining parts of the specification are all calculated in the same manner.

### (16) Stage 16

This is a process of extracting a cathode active material attached to the surface of a waste crucible by immersing the waste crucible in the valuable metal extractant produced in stage 15 after calcination of the cathode active material, and then growing nickel sulfate crystals using the extract containing the extracted valuable metals under the same method and conditions as the vertical temperature gradient method in stage 7.

That is, with regard to the nickel sulfate crystals grown using a vertical temperature gradient method, an extract containing valuable metals is introduced into a crystal growth tank, and a temperature control system is operated to maintain the temperature of an upper part of the crystal growth tank in the range of 15 to 20 °C and the temperature of a lower part thereof in the range of 2 to 4 °C, so that a temperature gradient (Δt) between the upper and lower parts of the crystal growth tank is set to 11 to 18 °C and maintained for 12 to 24 hours to thus obtain the nickel sulfate crystals.

### (17) Stage 17

This is a process of filtering and separating the nickel sulfate crystals obtained in stage 16 using a 60 to 100 mesh net and recovering the remaining filtrate.

### (18) Stage 18

This is a process of obtaining nickel sulfate powder by drying the nickel sulfate crystals filtered and separated in stage 17 at a temperature in the range of 60 to 80 °C for 10 to 12 hours.

### (19) Stage 19

This is a process of adding sodium hydroxide to the filtrate recovered in stage 17 to adjust the pH of the filtrate to 11 to 12, supplying carbon dioxide to adjust the pH to a range of 8 to 8.5, performing a precipitation reaction for 12 hours, followed by solid-liquid separation using a centrifuge to obtain solids and drying the solids at a temperature in the range of 90 to 110 °C for 12 hours, thereby producing lithium carbonate powder.

### (20) Stage 20

This is a process for producing a slurry type desalinating agent for salt removal by adding 25 to 40 wt. parts of an additive (B) to 60 to 75 wt. parts of a solution, in which 3 kg of sodium sulfate decahydrate crystals obtained in stage 9 is dissolved in 20 L of the usable water prepared in stage 10.

In this regard, the additive (B) is prepared by mixing 7 to 10 wt. parts of colloidal amorphous calcium carbonate slurry, 5 to 8 wt. parts of amorphous magnesium hydroxide slurry, 5 to 8 wt. parts of aluminum hydroxide powder, and 3 to 4 wt. parts of nanocarbon colloidal solution.

### (21) Stage 21

This is a process for producing a slurry type ammonia or hydrogen sulfide deodorant for odor removal by adding 20 to 30 wt. parts of an additive (C) to 70 to 80 wt. parts of a solution, in which 5 kg of sodium sulfate decahydrate crystals obtained in stage 9 is dissolved in 20 L of the usable water prepared in stage 10.

In this regard, when producing an ammonia deodorant, the additive (C) is prepared by mixing 3 kg of colloidal aluminum phosphate slurry, 1.3 kg of anatase-type titanium dioxide powder and 0.7 kg of nanocarbon colloidal solution. Further, when producing a hydrogen sulfide deodorant, the additive (C) is prepared by mixing 3 kg of amorphous magnesium hydroxide slurry, 1.3 kg of anatase-type titanium dioxide powder and 0.7 kg of nanocarbon colloidal solution.

### (22) Stage 22

This is a process for producing a slurry type color and heavy metal remover for dyeing wastewater by adding 30 to 40 wt. parts of an additive (D) to 60 to 70 wt. parts of a solution, in which 5 kg of sodium sulfate decahydrate crystals obtained in stage 9 is dissolved in 20 L of the usable water prepared in stage 10.

In this regard, the additive (D) is prepared by mixing 10 to 14 wt. parts of hollow silica powder, 8 to 10 wt. parts of calcined dolomite powder, 8 to 10 wt. parts of hydroxyapatite powder and 4 to 6 wt. parts of nanocarbon colloidal solution.

Hereinafter, specific examples according to the present invention will be described.

### (1) Example 1

Example 1 was implemented to produce sodium sulfate decahydrate crystals from high-concentration secondary battery salt wastewater by a vertical temperature gradient method based on an aqueous solution crystal growth method. The production of sodium sulfate decahydrate crystals was performed according to the diagram shown in FIG. 1.

Salt wastewater discharged from a process of producing precursors for secondary battery cathode materials generally has a sulfate ion concentration of 60,000 to 68,000 mg/L, a salinity of 5 to 6%, a pH of 9 to 10, and a nickel (Ni) heavy metal content of 400 to 450 mg/L.

Such salt wastewater is preheated to a temperature in the range of 30 to 40 °C in a preheater by circulating high-temperature steam discharged from a hydrothermal reactor, and then introduced into a horizontal pipe-type hydrothermal reactor maintained at a temperature of 120 to 150 °C, wherein a hydrothermal reaction is induced for 30 minutes to produce a sodium sulfate supersaturated solution having a salinity of 8 to 9%, which is then transported to a supersaturated solution storage tank.

Carbon dioxide is supplied to the sodium sulfate supersaturated solution having a pH range of 9 to 10, which was introduced into a storage tank, to induce a carbonation reaction until the pH reaches a range of 7 to 7.5, while conducting precipitation for 12 hours. Then, the precipitate is recovered using a centrifuge and is dried at 100°C for 12 hours to obtain nickel carbonate powder.

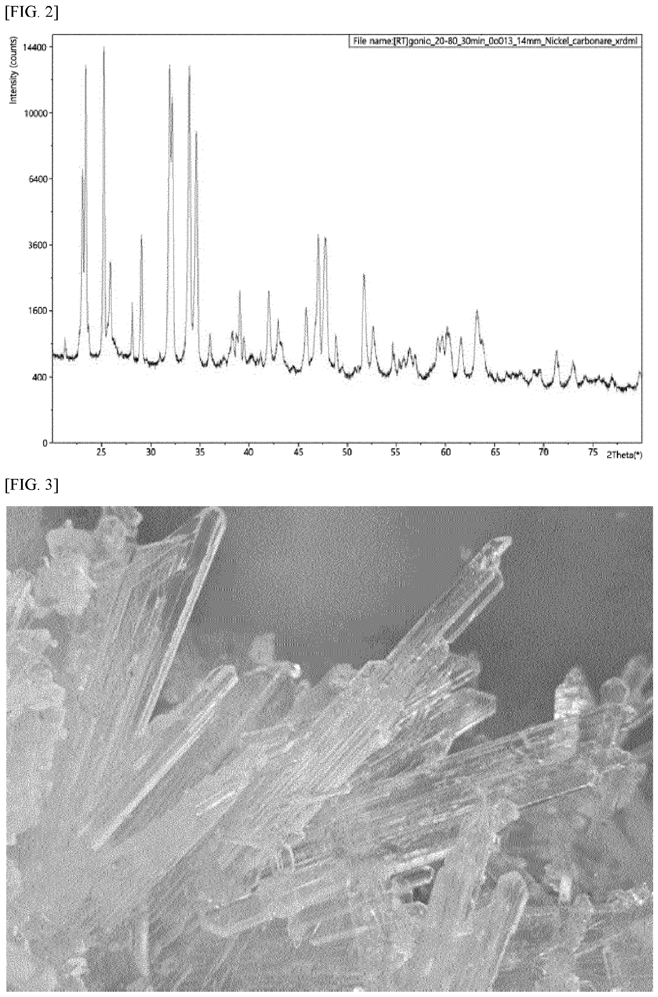

FIG. 2 shows the results of X-ray diffraction (XRD) analysis for nickel carbonate (NiCO₃) powder, and as a result of identification using the Joint Committee on Powder Diffraction Standard (JCPDS) card, it was confirmed that the peak pattern matched nickel carbonate.

Then, a fluid, in which the sodium sulfate supersaturated solution and carbon dioxide coexist, is introduced into an aqueous solution crystal growth tank that is configured to perform a vertical temperature gradient method.

A temperature control system is set to maintain an upper temperature of the aqueous solution crystal growth tank at 18 °C and a lower temperature thereof at 3 °C in the vertical temperature gradient method, such that a temperature gradient (Δt) between the upper and lower parts becomes 15 °C (a condition in which sodium ions and sulfate ions diffuse well), and crystal growth is induced for 24 hours.

Then, after separating the crystals and the filtrate using a 100 mesh net, a natural drying process is performed to obtain sodium sulfate decahydrate crystals as shown in FIG. 3. The filtrate obtained herein is cold water below 10 °C and mixed with steam from the preheater, and then reused as the usable water required in a resource recovery process.

As a result of test analysis conducted by the Joint Laboratory Practice Center of Kyungpook National University for analysis of nickel heavy metal content in a target water for treatment, that is, salt wastewater (raw water), and treated water (usable water), the nickel content in the salt wastewater (raw water) was 440.3 mg/L whereas no nickel was detected in the treated water (usable water), as shown in Table 1.

**[TABLE 1]**

| Test item | Raw water (salt wastewater) | Treated water (usable water) |
|---|---|---|
| Nickel (Ni) | 440.3 mg/L | ND (Not detected) |

Further, the analysis of nickel heavy metal content in the sodium sulfate decahydrate crystals and sodium sulfate powder obtained in Example 1 was requested from the Joint Laboratory Practice Center of Kyungpook National University for test analysis, and as shown in Table 2, the result was determined as not detected (ND).

**[TABLE 2]**

| Test item | Sodium sulfate decahydrate crystals | Sodium sulfate powder |
|---|---|---|
| Nickel (Ni) | ND (Not detected) | ND (Not detected) |

### (2) Example 2

Example 2 shows the results of resource recovery using sodium sulfate decahydrate crystals. The resource recovery process using sodium sulfate decahydrate crystals is performed according to the process diagram of FIG. 4.

In Example 2, 0.5 L (liter) of a 35% hydrochloric acid solution was added to 20 L (liter) of the usable water prepared using the filtrate discharged in Example 1, 10 kg of sodium sulfate decahydrate crystals and 60 g of ethylene diamine tetraacetic acid for promoting the extraction of cathode active materials were introduced thereto, followed by stirring at room temperature and at a stirring speed of 100 rpm for 1 hour to completely dissolve the solution, thereby obtaining a liquid-type valuable metal extractant.

As a result of immersing a waste crucible after calcination of a cathode active material in such a valuable metal extractant for 24 hours, the cathode active material attached to the surface of the waste crucible is extracted, as shown in FIG. 5. When the extraction of the cathode active material is completed, the waste crucible is separated, and the remaining extract containing valuable metals is used to produce nickel sulfate powder and lithium carbonate powder.

In this example, nickel sulfate crystals and a filtrate are obtained from the extract containing valuable metals using the vertical temperature gradient method in the same manner and under the same conditions as in Example 1, nickel sulfate crystals are filtered and separated using a 100 mesh net, and the filtered and separated nickel sulfate crystals are dried at 80 °C for 12 hours, thereby obtaining nickel sulfate powder.

Then, after the filtrate is adjusted to pH 12 by adding sodium hydroxide, carbon dioxide is supplied thereto for a carbonation reaction until the pH becomes 8. Thereafter, the filtrate is subjected to a precipitation reaction at room temperature for 12 hours, solid-liquid separation using a centrifuge, followed by drying the solids at 100 °C for 12 hours to thus obtain lithium carbonate powder.

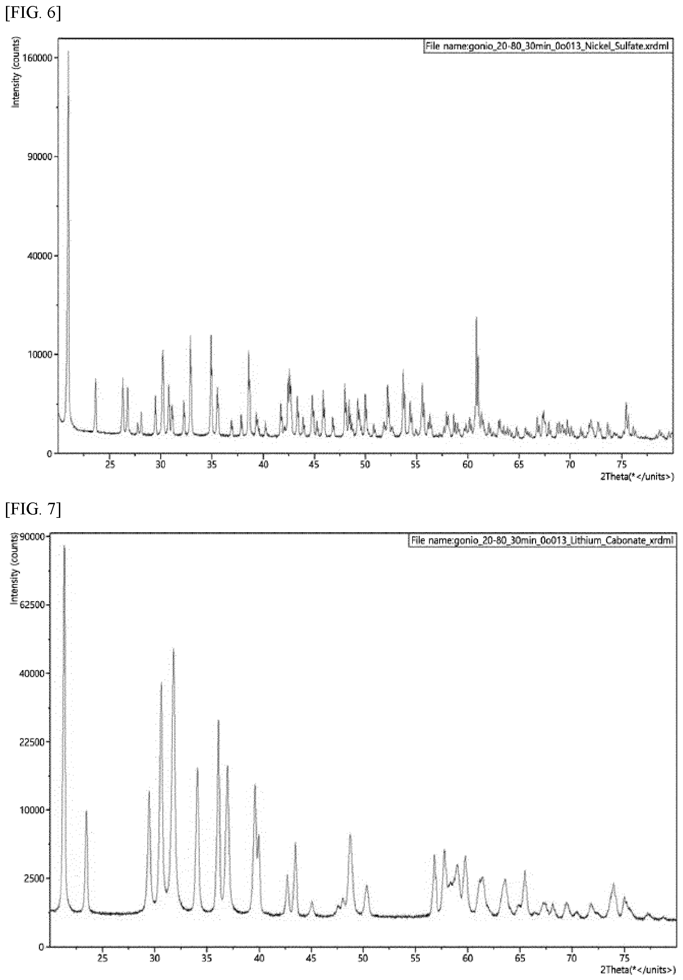

FIGS. 6 and 7 show the results of X-ray diffraction analysis for nickel sulfate powder and lithium carbonate powder, respectively. As a result of identification using the JCPDS cards, it was confirmed that the peak patterns matched those of nickel sulfate and lithium carbonate, respectively.

Although sulfuric acid solutions have been used in most conventional valuable metal extraction, it could be confirmed from the results of Example 2 that the valuable metal extractant according to the present invention can replace the sulfuric acid solution.

### (3) Example 3

In Example 3, a desalinating agent for salt removal is produced using sodium sulfate decahydrate crystals and the usable water prepared in Example 1.

In Example 3, 3 kg of the sodium sulfate decahydrate crystals obtained in Example 1 were added to 20 L (liters) of usable water, stirred at room temperature and at a stirring speed of 100 rpm for 1 hour to completely dissolve the solution. Then, 6 kg of an additive (2 kg of colloidal amorphous calcium carbonate slurry, 1.5 kg of amorphous magnesium hydroxide slurry, 1.5 kg of aluminum hydroxide powder, and 1 kg of nanocarbon colloidal solution) was mixed with 14 kg of the above solution to produce a desalinating agent in the form of a slurry in a 20 kg packaging unit.

In review of examples of field application of a desalinating agent, in the case of reclaimed land in Gwanghwal-myeon, Gimje-si, Jeollabuk-do, which is owned by 365 Agricultural Cooperative Association, the average soil salt concentration was 1.35%. As a result of first desalination treatment of the soil with a 50-fold dilution of the desalinating agent, the salt concentration decreased to 0.41%. Further, 3 days later, as a result of second desalination treatment of the soil with a 100-fold dilution of the desalinating agent, the salt concentration decreased to 0.02%. These test analysis results are shown in Table 3.

**[TABLE 3]**

| Division | Salt concentration | Removal rate |
|---|---|---|
| Soil salinity | 1.35 % | - |
| First desalination | 0.41 % | 69.6 % |
| Second desalination | 0.02 % | 98.5 % |

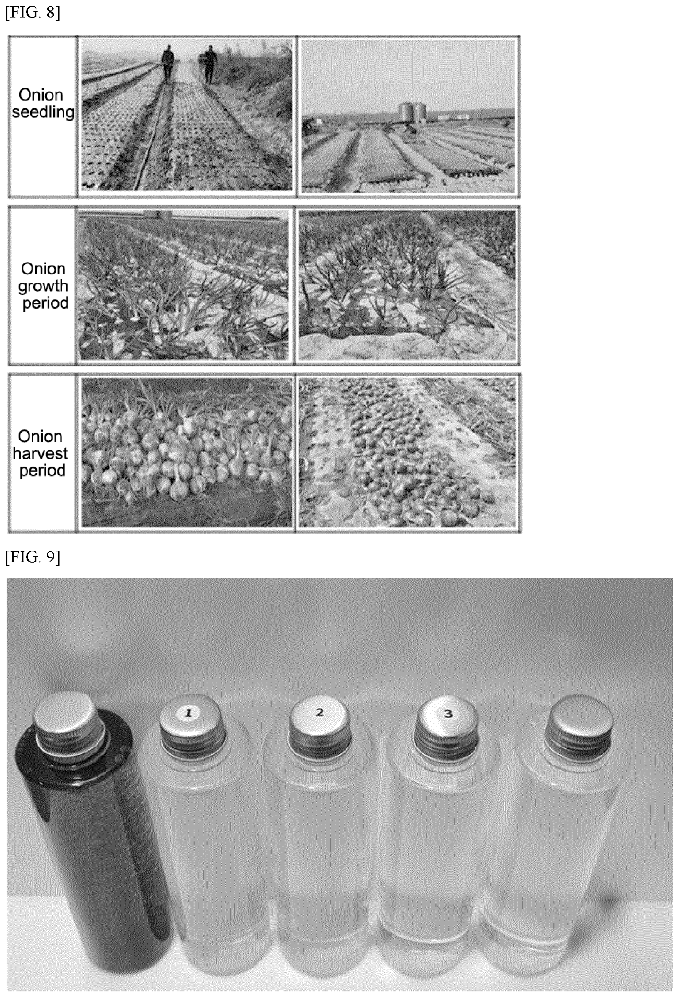

Generally, it is reported that the salt concentration of soil suitable for crop cultivation is 0.3% or less. Therefore, in Example 3, onions as a cash crop were successfully cultivated and harvested in cooperation with 365 Agricultural Cooperative Association, as shown in FIG. 8.

### (4) Example 4

In Example 4, an ammonia or hydrogen sulfide deodorant is produced using sodium sulfate decahydrate crystals. In Example 4, the usable water prepared in Example 1 is used as a solution required for producing the ammonia or hydrogen sulfide deodorant.

In Example 4, 5 kg of the sodium sulfate decahydrate crystals obtained in Example 1 were added to 20 L (liters) of usable water, stirred at room temperature for 1 hour at a stirring speed of 100 rpm to completely dissolve the solution. Then, 5 kg of an additive was mixed with 15 kg of the above solution to produce a slurry type ammonia or hydrogen sulfide deodorant for odor removal in a 20 kg packaging unit.

In this regard, an additive for the ammonia deodorant is prepared by mixing 3 kg of colloidal aluminum phosphate slurry, 1.3 kg of anatase-type titanium dioxide powder and 0.7 kg of nanocarbon colloidal solution, while an additive for the hydrogen sulfide deodorant is prepared by mixing 3 kg of amorphous magnesium hydroxide slurry, 1.3 kg of anatase-type titanium dioxide powder, and 0.7 kg of nanocarbon colloidal solution.

**[TABLE 4]**

| Test item | Elapsed time | Deodorization rate |
|---|---|---|
| Ammonia deodorization test | After 30 minutes | 98.5% or more |
| Hydrogen sulfide deodorization test | After 30 minutes | 99.0% or more |

The results of the deodorization test on ammonia or hydrogen sulfide deodorants requested from the Korea Testing & Research Institute for Chemical Industry are summarized in Table 4. As a result, it was determined that the ammonia deodorization rate was 98.5% or more and the hydrogen sulfide deodorization rate was 99.0% or more after 30 minutes.

### (5) Example 5

In Example 5, a color and heavy metal remover for dyeing wastewater is prepared using sodium sulfate decahydrate crystals. In Example 5, the usable water prepared in Example 1 is used as a solution required for the preparation of the color and heavy metal remover for dyeing wastewater.

In Example 5, 5 kg of the sodium sulfate decahydrate crystals obtained in Example 1 were added to 20 L (liters) of usable water, stirred at room temperature for 1 hour at a stirring speed of 100 rpm to completely dissolve the solution. Then, 7 kg of an additive (2.3 kg of hollow silica powder, 2 kg of calcined dolomite powder, 2 kg of hydroxyapatite powder and 0.7 kg of nanocarbon colloidal solution) was mixed with 13 kg of the above solution to produce a slurry type color and heavy metal remover for dyeing wastewater in a 20 kg packaging unit.

Using Example 5, the dyeing wastewater discharged from J Industrial Complex in Pocheon-si, Gyeonggi-do was subjected to the removal of color and heavy metals, and it could be confirmed in FIG. 9 that the color of the dyeing wastewater decreased from the left to the right. Further, as a result of requesting the Korea Environment Corporation to test and analyze the color and heavy metal contents of dyeing wastewater (raw water) and treated water, it was determined that the color removal rate was 84.8% and the heavy metal removal rate was 100%. The results of such test analysis are as shown in Tables 5 and 6.

**[TABLE 5]**

| Test item | Dyeing wastewater (raw water) | Treated water |
|---|---|---|
| Color | 204 degrees | 31 degrees |

**[TABLE 6]**

| Test item | Dyeing wastewater (raw water) | Treated water |
|---|---|---|
| Copper (Cu) | 0.099 mg/L | Not detected |
| Chromium (Cr) | 0.369 mg/L | Not detected |
| Lead (Pb) | Not detected | - |
| Arsenic (As) | Not detected | - |
| Cadmium (Cd) | Not detected | - |

### (6) Example 6

In Example 6, sodium sulfate powder is produced using sodium sulfate decahydrate crystals. In Example 6, sodium sulfate powder is obtained by drying sodium sulfate decahydrate crystals at 250 °C for 30 minutes.

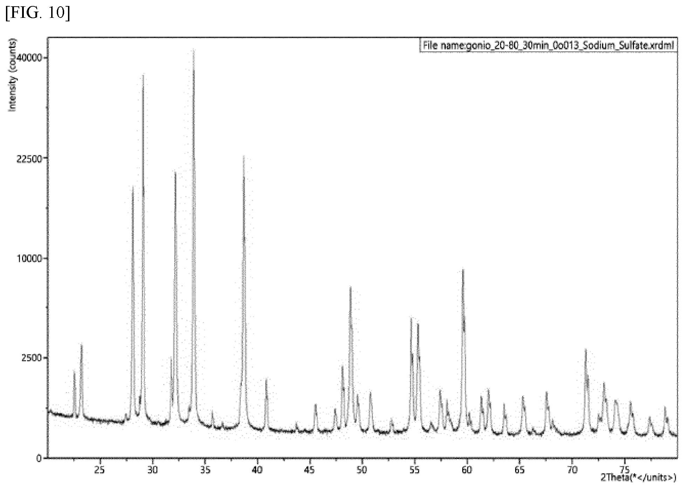

FIG. 10 shows the results of X-ray diffraction analysis for sodium sulfate powder. The peak pattern was identified using a JCPDS cards and was confirmed to be consistent with sodium sulfate.

### (7) Example 7

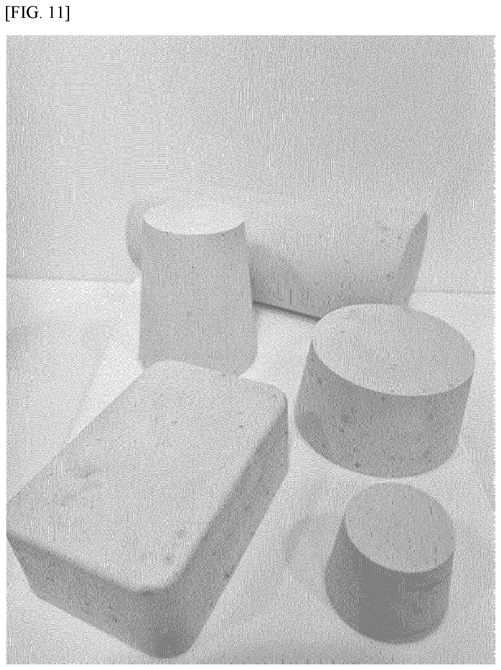

In Example 7, an ammonia nitrogen remover for water purification is produced using sodium sulfate powder. 7.5 kg of sodium sulfate powder and 2.5 kg of an additive (1.3 kg of non-swelling mica powder, 0.6 kg of calcium aluminate powder, 0.4 kg of nanocarbon colloidal solution and 0.2 kg of silver (Ag) colloidal solution) are mixed to prepare 10 kg of a mixture, and 2 kg of recycled water is added to the mixture to thus prepare a slurry, which is then injected into various molds and dried naturally to produce ammonia nitrogen removers (carrier type) with different sizes and shapes, as shown in FIG. 11.

As a result of requesting Korea E&C Co., Ltd. to conduct a test analysis of ammonia nitrogen for an ammonia nitrogen remover (a carrier type), the removal rate of ammonia nitrogen was 62.4%, and the test analysis results are shown in Table 7.

**[TABLE 7]**

| Test item | Removal rate |
|---|---|
| Ammonia nitrogen | 62.4% |

Although specific embodiments of the present invention have been described above with reference to the accompanying drawings, the scope of protection of the present invention is not limited thereto. It will be apparent that various modifications, additions or deletions of known techniques, simple numerical modifications, and the like may be made without departing from the technical spirit of the present invention, and such modifications are also included within the scope of protection of the present invention.

## Claims

1. A method for producing sodium sulfate decahydrate crystals from high-concentration secondary battery salt wastewater by an aqueous solution crystal growth method, and for resource recovery using the prepared crystals, comprising:
Stage 1 of transporting high-concentration secondary battery salt wastewater to a preheater;
Stage 2 of heating the salt wastewater introduced into the preheater to 30 to 40 °C by supplying high-temperature steam (high-temperature vapor) discharged from a hydrothermal reactor to the preheater;
Stage 3 of transporting the salt wastewater preheated in stage 2 to a horizontal pipe-type hydrothermal reactor and then performing a hydrothermal reaction that heats the salt wastewater to 120 to 150 °C for 30 to 60 minutes in order to evaporate moisture until a salinity of the introduced salt wastewater reaches 8 to 9%, thereby producing a sodium sulfate supersaturated solution;
Stage 4 of feeding the high-temperature steam discharged in stage 3 to the preheater in stage 2, and transporting the sodium sulfate supersaturated solution with the salinity of 8 to 9% to a supersaturated solution storage tank;
Stage 5 of supplying carbon dioxide to the sodium sulfate supersaturated solution flowing into the supersaturated solution storage tank in order to adjust the pH to a range of 7 to 7.5 while precipitating nickel carbonate (NiCO₃), followed by separating and recovering the precipitate so as to remove nickel heavy metals contained in the sodium sulfate supersaturated solution;
Stage 6 of transporting the sodium sulfate supersaturated solution with the pH adjusted to 7 to 7.5 and carbon dioxide to an aqueous solution crystal growth tank;
Stage 7 of performing a vertical temperature gradient method, which controls an upper temperature of the aqueous solution crystal growth tank to a temperature in the range of 15 to 20 °C and a lower temperature thereof to a temperature in the range of 2 to 4 °C, for 12 to 24 hours, thereby growing and obtaining sodium sulfate decahydrate crystals;
Stage 8 of filtering and separating the sodium sulfate decahydrate crystals obtained in stage 7 using a 60 to 100 mesh net and recovering the remaining filtrate;
Stage 9 of naturally drying the sodium sulfate decahydrate crystals filtered and separated in stage 8; and
Stage 10 of mixing the steam discharged from the preheater in stage 2 with the filtrate recovered by filtering and separating the sodium sulfate decahydrate crystals in stage 8, so as to produce usable water to be reused in a resource recovery process.

2. The method according to claim 1, further comprising:
Stage 11 of obtaining sodium sulfate powder by drying the sodium sulfate decahydrate crystals obtained in stage 9 at a high temperature in a range of 200 to 300 °C for 20 to 30 minutes;
Stage 12 of adding 15 to 20 parts by weight ('wt. parts') of the usable water prepared in stage 10 and 20 to 30 wt. parts of an additive (E) to 70 to 80 wt. parts of the sodium sulfate powder obtained in stage 11, and mixing them;
Stage 13 of molding the mixture in a slurry state obtained in stage 12; and
Stage 14 of producing an ammonia nitrogen remover for water purification in various forms by naturally drying a molded product formed in stage 13,
wherein the additive (E) is prepared by mixing 10 to 15 wt. parts of non-swelling mica powder, 5 to 7 wt. parts of calcium aluminate powder, 3 to 5 wt. parts of nanocarbon colloidal solution, and 2 to 3 wt. parts of silver (Ag) colloidal solution.

3. The method according to claim 1, further comprising:
Stage 15 of producing a valuable metal extractant by dissolving 10 kg of sodium sulfate decahydrate crystals obtained in stage 9 in 20 L of the usable water prepared in stage 10 and then adding an additive (A) thereto,
wherein the additive (A) is prepared by mixing 0.5 L of a 35% concentration hydrochloric acid solution and 0.06 kg of ethylene diamine tetraacetic acid (EDTA).

4. The method according to claim 3, further comprising:
Stage 16 of extracting a cathode active material attached to the surface of a waste crucible by immersing the waste crucible in the valuable metal extractant produced in stage 15 after calcination of the cathode active material, and then growing nickel sulfate crystals using the extract containing extracted valuable metals, under the same method and conditions as the vertical temperature gradient method in stage 7;
Stage 17 of filtering and separating the nickel sulfate crystals obtained in stage 16 using a 60 to 100 mesh net and recovering the remaining filtrate; and
Stage 18 of obtaining nickel sulfate powder by drying the nickel sulfate crystals filtered and separated in stage 17 at a temperature in the range of 60 to 80 °C for 10 to 12 hours.

5. The method according to claim 4, further comprising:
Stage 19 of adding sodium hydroxide to the filtrate recovered in stage 17 to adjust the pH of the filtrate to 11 to 12, supplying carbon dioxide to adjust the pH to a range of 8 to 8.5, performing a precipitation reaction for 12 hours, followed by solid-liquid separation using a centrifuge to obtain solids and drying the solids at a temperature in the range of 90 to 110 °C for 12 hours to thus produce lithium carbonate powder.

6. The method according to claim 1, further comprising:
Stage 20 of producing a slurry type desalinating agent for salt removal by adding 25 to 40 wt. parts of an additive (B) to 60 to 75 wt. parts of a solution, in which 3 kg of sodium sulfate decahydrate crystals obtained in stage 9 is dissolved in 20 L of the usable water prepared in stage 10,
wherein the additive (B) is prepared by mixing 7 to 10 wt. parts of colloidal amorphous calcium carbonate slurry, 5 to 8 wt. parts of amorphous magnesium hydroxide slurry, 5 to 8 wt. parts of aluminum hydroxide powder, and 3 to 4 wt. parts of nanocarbon colloidal solution.

7. The method according to claim 1, further comprising:
Stage 21 of producing a slurry type ammonia or hydrogen sulfide deodorant for odor removal by adding 20 to 30 wt. parts of an additive (C) to 70 to 80 wt. parts of a solution, in which 5 kg of sodium sulfate decahydrate crystals obtained in stage 9 is dissolved in 20 L of the usable water prepared in stage 10,
wherein, when producing an ammonia deodorant, the additive (C) is prepared by mixing 3 kg of colloidal aluminum phosphate slurry, 1.3 kg of anatase-type titanium dioxide powder and 0.7 kg of nanocarbon colloidal solution, and
wherein, when producing a hydrogen sulfide deodorant, the additive (C) is prepared by mixing 3 kg of amorphous magnesium hydroxide slurry, 1.3 kg of anatase-type titanium dioxide powder and 0.7 kg of nanocarbon colloidal solution.

8. The method according to claim 1, further comprising:
Stage 22 of producing a slurry type color and heavy metal remover for dyeing wastewater by adding 30 to 40 wt. parts of an additive (D) to 60 to 70 wt. parts of a solution, in which 5 kg of sodium sulfate decahydrate crystals obtained in stage 9 is dissolved in 20 L of the usable water prepared in stage 10,
wherein the additive (D) is prepared by mixing 10 to 14 wt. parts of hollow silica powder, 8 to 10 wt. parts of calcined dolomite powder, 8 to 10 wt. parts of hydroxyapatite powder and 4 to 6 wt. parts of nanocarbon colloidal solution.
